# EUROPÄISCHE PATENTSCHRIFT

(11) **EP 0 564 810 B1**
(45) Veröffentlichungstag und Bekanntmachung des Hinweises auf die Patenterteilung: **02.11.1994**
(21) Anmeldenummer: 93103218.9
(22) Anmeldetag: 01.03.1993
(51) Int. Cl.: C07D 249/08, C07D 303/08, C07D 303/18, A01N 43/653

(54) **Fungizide Azolylmethyl-fluorcycloproplylderivate**
Fungicidal azolylmethyl-fluorocyclopropyl derivatives
Dérivés azolylméthyl-fluorocyclopropiliques fongicides

(30) Priorität: 13.03.1992 DE 4208050
(43) Veröffentlichungstag der Anmeldung: 13.10.1993
(73) Patentinhaber: BAYER AG, 51368 Leverkusen (DE)
(72) Erfinder: Scherkenbeck, Jürgen, Dr., W-5090 Leverkusen 1 (DE); Himmler, Thomas, Dr., W-5068 Odenthal (DE); Böhm, Stefan, Dr., W-5000 Köln 80 (DE); Hagemann, Hermann, Dr., W-5090 Leverkusen 1 (DE); Stroech, Klaus, Dr., W-5650 Solingen 19 (DE); Dutzmann, Stefan, Dr., W-4010 Hilden 1 (DE); Dehne, Heinz-Wilhelm, Dr., W-4019 Monheim (DE); Hänssler, Gerd, Dr., W-5090 Leverkusen 3 (DE)

(56) Entgegenhaltungen:
- EP-A- 0 047 594
- EP-A- 0 080 102
- EP-A- 0 297 345

## Beschreibung

Die vorliegende Erfindung betrifft neue Azolylmethylfluorcyclopropyl-Derivate, ein Verfahren zu deren Herstellung und deren Verwendung als Fungizide.

Es ist bereits bekannt geworden, daS bestimmte Azolylmethyl-cyclopropyl-Derivate fungizide Eigenschaften besitzen (vergleiche EP-OS 0 297 345). So kann z.B. 1-(4-Chlorphenyl)-2-(1-fluor-cyclopropyl)-3-(1,2,4-triazol-1-yl)-propan-2-ol zur Bekämpfung von Pilzen eingesetzt werden. Die Wirkung dieses Stoffes ist gut, laßt aber bei niedrigen Aufwandmengen in manchen Fällen zu wünschen übrig.

Es wurden nun neue Azolylmethyl-fluorcylopropyl-Derivate der Formel 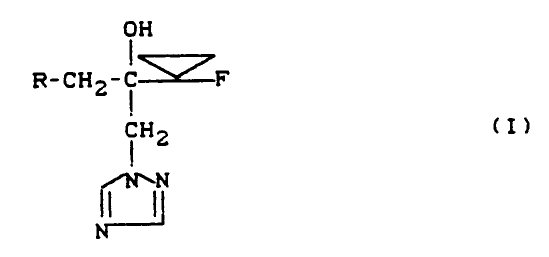
in welcher
R für 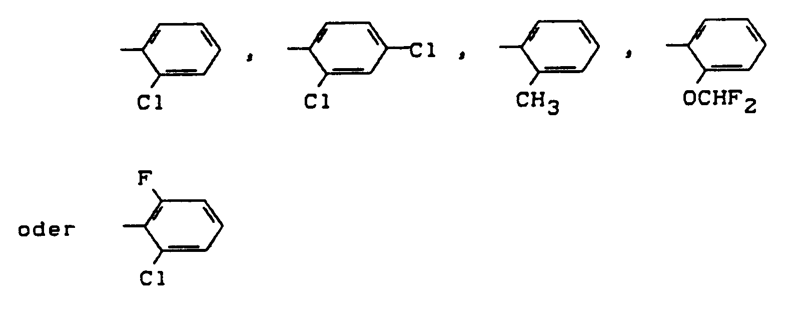
steht,
sowie deren Säureadditions-Salze und Metallsalz-Komplexe gefunden.

Weiterhin wurde gefunden, daß man Azolylmethyl-fluorcyclopropyl-Derivate der Formel (I) sowie deren Säureadditions-Salze und Metallsalz-Komplexe erhält, wenn man Oxirane der Formel 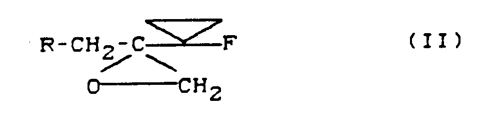
in welcher
R die oben angegebene Bedeutung hat,
mit 1,2,4-Triazol der Formel 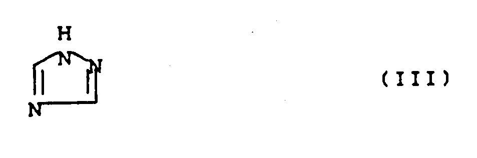
gegebenenfalls in Gegenwart eines Säurebindemittels und in Gegenwart eines Verdünnungsmittels umsetzt, und gegebenenfalls anschließend an die so erhaltenen Verbindungen der Formel (I) eine Säure oder ein Metallsalz addiert.

Schließlich wurde gefunden, daß die neuen Azolylmethylfluorcyclopropyl-Derivate der Formel (I) sowie deren Säureadditions-Salze und Metallsalz-Komplexe sehr gute fungizide Eigenschaften aufweisen.

Die erfindungsgemäßen Stoffe enthalten ein asymmetrisch substituiertes Kohlenstoffatom. Sie Können daher in optischen Isomerenformen anfallen. Die vorliegende Erfindung betrifft sowohl die einzelnen Isomeren als auch deren Gemische.

Überraschenderweise besitzen die erfindungsgemäßen Stoffe bessere fungizide Eigenschaften als das 1-(4-Chlorphenyl)-2-(1-fluor-cyclopropyl)-3-(1,2,4-triazol-1-yl)-propan-2-ol, welches der konstitutionell ähnlichste vorbekannte Wirkstoff gleicher Wirkungsrichtung ist.

Bevorzugte Säureadditions-Salze erfirdungsgemäßer Stoffe sind solche, die durch Addition folgender Säuren an Azolylmethyl-fluorcyclopropyl-Derivate der Formel (I) entstehen:
Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure, Saccharin und Thiosaccharin.

Bevorzugte Metallsalz-Komplexe erfindungsgemäßer Stoffe sind Additionsprodukte aus Salzen von Metallen der II. bis IV. Haupt- und der I. und II. sowie IV. bis VIII. Nebengruppe des Periodensystems der Elemente und Azolylmethyl-fluorcyclopropyl-Derivaten der Formel (I). Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-(2-Chlorbenzyl)-2-(1-fluor-cyclopropyl)-oxiran als Ausgangsstoff und 1,2,4-Triazol als Reaktionskomponente, so kann der Verlauf des erfindungsgemäßen Verfahrens durch das folgende Formelschema veranschaulicht werden: 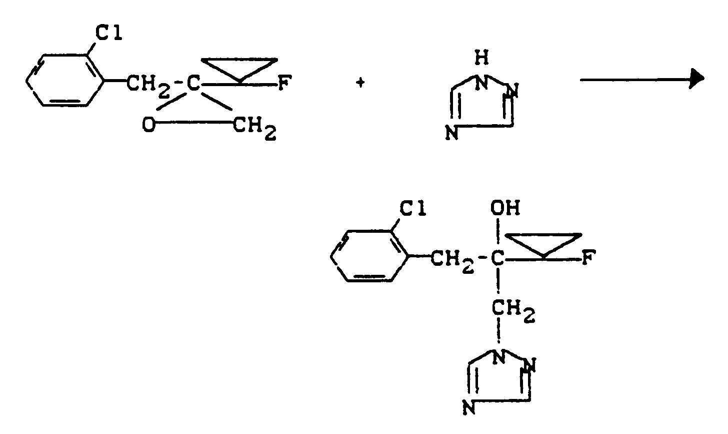
Die bei dem erfindungsgemäßen Verfahren als Ausgangsstoffe benötigten Oxirane der Formel (II) sind bisher noch nicht bekannt. Sie lassen sich herstellen, indem man Benzylketone der Formel 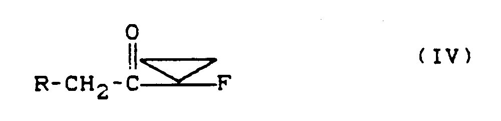
in welcher
R die oben angegebene Bedeutung hat, entweder
α) mit Dimethyloxosulfonium-methylid der Formel 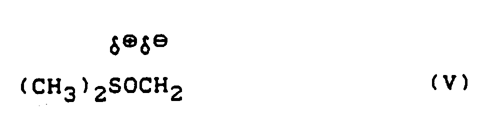 oder
β) mit Dimethylsulfonium-methylid der Formel 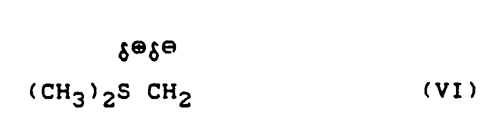 in Gegenwart eines Verdünnungsmittels umsetzt.

Die Benzylketone der Formel (IV) lassen sich herstellen, indem man
a) in einer ersten Stufe Benzylchloride der Formel

   R-CH₂-Cl (VII)

   in welcher
   R die oben angegebene Bedeutung hat,
   mit einem Überschuß an Zinkpulver in Gegenwart eines Verdünnungsmittels, wie z.B. Ethylenglykoldimethylether, bei Temperaturen zwischen 50°C und 150°C unter Schutzgasatmosphäre umsetzt, das überschüssige Zinkpulver abtrennt und dann
b) in einer zweiten Stufe die entstandenen Benzylderivate der Formel

   R-CH₂-ZnCl (VIII)

   in welcher
   R die oben angegebene Bedeutung hat,
   mit 1-Fluor-cyclopropan-carbonsäurechlorid der Formel 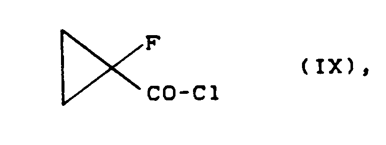 in Gegenwart eines Palladiumkatalysators, wie z.B. Bis-(triphenyl-phosphin)-palladium-(II)-chlorid, und in Gegenwart eines Verdünnunsmittels, wie z.B. Ethylenglykol-dimethylether, bei Temperaturen zwischen 20°C und 100°C umsetzt.

Die Verbindungen der Formeln (VII) und (IX) sind bekannt oder lassen sich nach prinzipiell bekannten Verfahren herstellen (vergleiche EP-OS 0 436 348 und EP-OS 0 461 483).

Das bei der Durchführung der Variante (α) des Verfahrens zur Herstellung von Oxiranen der Formel (II) als Reaktionskomponente benötigte Dimethyloxosulfonium-methylid der Formel (V) ist bekannt (vergleiche J. Am. Chem. Soc, 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, insbesondere mit Kalium-tert.-butylat oder Natriummethylat, oder durch Umsetzung von Trimethyloxosulfoniumchlorid mit wäßriger Natronlauge, jeweils in Gegenwart eines Verdünnungsmittels erzeugt.

Das bei der Durchführung der Variante (β) des Verfahrens zur Herstellung von Oxiranen der Formel (II) außerdem als Reaktionskomponente in Betracht kommende Dimethylsulfonium-methylid der Formel (VI) ist ebenfalls bekannt (vgl. Heterocycles 8, 397 (1977)). Es wird bei der obigen Umsetzung ebenfalls in frisch hergestelltem Zustand eingesetzt, indem man es in situ z.B. aus Trimethylsulfonium-halogenid oder Trimethylsulfonium-methylsulfat, in Gegenwart einer starken Base, wie z.B. Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des obigen Verfahrens zur Herstellung von Oxiranen der Formel (II) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid.

Die Reaktionstemperaturen können bei der Durchführung des obigen Verfahrens zur Herstellung von Oxiranen der Formel (II) in einem größeren Bereich variiert werden. Im allgemeinen arbeitet man bei Temperaturen zwischen 0°C und 100°C, vorzugsweise zwischen 10°C und 60°C.

Bei der Durchführung des obigen Verfahrens zur Herstellung von Oxiranen der Formel (II) setzt man auf 1 Mol an Benzylketon der Formel (IV) im allgemeinen 1 bis 3 Mol an Dimethyloxosulfonium-methylid der Formel (V) bzw. an Dimethylsulfonium-methylid der Formel (VI) ein. Die Isolierung der Oxirane der Formel (II) erfolgt nach üblichen Methoden.

Als Säurebindemittel kommen bei der Durchführung des erfindungsgemäßen Verfahrens alle üblichen anorganischen und organischen Basen in Frage. Vorzugsweise verwendbar sind Alkalimetallcarbonate, wie Natrium- und Kaliumcarbonat, ferner Alkalimetallhydroxide, wie Natrium- und Kaliumhydroxid, außerdem Alkalimetallalkoholate, wie Natrium-und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 50°C und 150°C.

Bei der Durchführung des erfindungsgemäßen Verfahrens setzt man vorzugsweise auf 1 Mol an Oxiran der Formel (II) 1 bis 4 Mol an Azol der Formel (III) und 1 bis 2 Mol an Base ein. Die Isolierung der Endprodukte erfolgt in üblicher Weise.

Die erfindungsgemäßen Azolylmethyl-fluorcyclopropyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brausicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Leptosphaeria nodorum, Cochliobolus sativus, Pyrenophora teres, Pseudocercosporella herpotrichoides, Erysiphe und Fusarium-Arten. Außerdem zeigen die erfindungsgemäßen Stoffe eine sehr gute Wirkung gegen Venturia, Sphaerotheca und Botrytis.

Die erfindungsgemäßen Stoffe Können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet worden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g benötigt. Bei der Behandlung des Bodens sind Wirkstoffkonzentrationen von 0,00001 bis 0,1 Gew.-%, vorzugsweise von 0,0001 bis 0,02 %, am Wirkungsort erforderlich.

Die Herstellung und die Verwendung der erfindungsgemäßen Stoffe geht aus den folgenden Beispielen hervor.

### Herstellungsbeispiele

### Beispiel 1

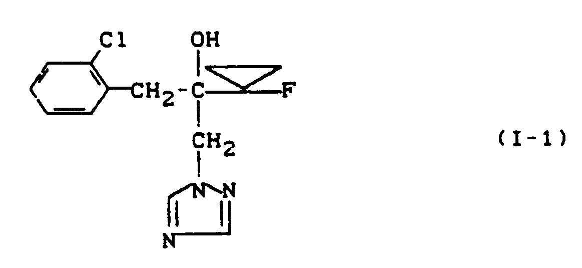

Eine Lösung von 5,33 g (23,51 mmol) 2-(2-Chlorbenzyl)-2-(1-fluorcyclopropyl)-oxiran in 15 ml Dimethylformamid wird bei 80°C unter Rühren in ein Gemisch aus 4,87 g (70,54 mmol) 1,2,4-Triazol und 0,53 g (4,70 mmol) Kalium-tert.-butylat in 20 ml Dimethylformamid eingetropft. Nach beendeter Zugabe wird das Reaktionsgemisch 6 Stunden bei 80°C gerührt. Anschließend wird das Lösungsmittel unter vermindertem Druck abgezogen, und der verbleibende Rückstand wird in Ethylacetat-Wasser aufgenommen. Die wäßrige Phase wird dreimal mit Ethylacetat extrahiert, und die vereinigten organischen Extrakte werden einmal mit Wasser gewaschen. Nach Trocknen der organischen Phase über Natriumsulfat und Einengen unter vermindertem Druck wird der Rückstand mit dem Laufmittel Ethylacetat: Cyclohexan = 1:2 bis 1:1 an Kieselgel chromatographiert. Nach dem Eindampfen des Eluates erhält man 4,3 g (62 % der Theorie) an 1-(2-Chlorphenyl)-2-(1-fluorcyclopropyl)-3-(1,2,4-triazol-1-yl)-propan-2-ol in Form einer Festsubstanz mit dem Schmelzpunkt 99 bis 103°C.

### Herstellung der Ausgangssubstanz:

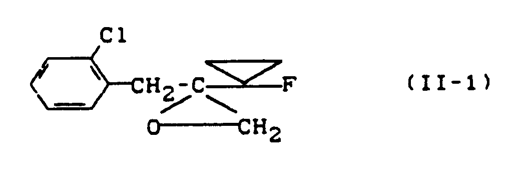

In ein Gemisch aus 3,33 g (25,86 mmol) Trimethylsulfoxoniumchlorid und 5,0 g (23,51 mmol) 2-Chlorbenzyl-(1-fluorcyclopropyl)-keton in 30 ml Toluol tropft man bei Raumtemperatur innerhalb einer Stunde 10,3 ml 45%ige wäßrige Natronlauge ein. Nach beendeter Zugabe wird eine Stunde bei Raumtemperatur nachgerührt. Das Reaktionsgemisch wird mit etwas Wasser verdünnt, und die organische Phase wird abgetrennt. Die wäßrige Phase wird dreimal mit Cyclohexan extrahiert. Die vereinigten organischen Extrakte werden über Natriumsulfat getrocknet und unter vermindertem Druck eingeengt. Der Rückstand wird ohne zusätzliche Reinigung für die weitere Umsetzung verwendet. 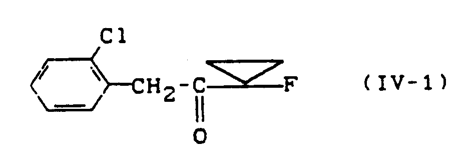
Ein Gemisch aus 32,7 g (0,5 Mol) Zinkpulver, 56,4 g (0,35 Mol) 2-Chlorbenzylchlorid und 375 ml trockenem Ethylenglykol-dimethylether wird unter Stickstoffatmosphäre 2 Stunden unter Rückfluß erhitzt. Anschließend wird das Reaktionsgemisch unter Stickstoff filtriert.

Das Filtrat wird mit 36,7 g (0,3 Mol) 1-Fluorcyclopropan-carbonsäurechlorid und 21 mg (0,01 Mol-%) Bis(triphenylphosphin)-palladium(II)-chlorid versetzt und unter Stickstoffatmosphäre 2 Stunden unter Rückfluß erhitzt. Nach dem Abkühlen auf Raumtemperatur wird das Reaktionsgemischt filtriert und unter vermindertem Druck eingeengt. Man nimmt den Rückstand in Toluol auf, schüttelt mit verdünnter, wäßriger Salzsäure aus, trocknet die organische Phase und zieht das Lösungsmittel unter vermindertem Druck ab. Der verbleibende Rückstand wird einer fraktionierten Destillation unterworfen. Dabei erhält man 55,4 g eines Öles, das gemäß Gaschromatogramm zu 90% aus 2-Chlorbenzyl-(1-fluor-cyclopropyl)-keton besteht. Die Ausbeute errechnet sich danach zu 75% der Theorie.

### Herstellung der Vergleichssubstanz der Formel

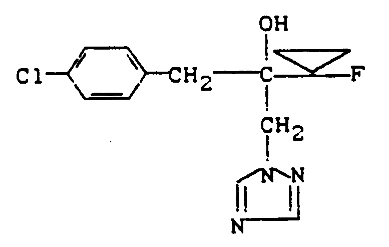

Eine Lösung von 3.3 g (15,7 mmol) 2-(4-Chlorbenzyl)-2-(1-fluorcyclopropyl)-oxiran in 10 ml Dimethylformamid wird bei 80°C unter Rühren in ein Gemisch aus 3,3 g (47,1 mmol) 1,2,4-Triazol und 0,35 g (3,14 mmol) Kalium-tert.-butylat in 20 ml Dimethylformamid eingetropft. Nach beendeter Zugabe wird das Reaktionsgemisch 13 Stunden bei 80°C gerührt. Anschließend wird das Lösungsmittel unter vermindertem Druck abgezogen, und der verbleibende Rückstand wird in Wasser aufgenommen. Die wäßrige Phase wird viermal mit Dichlormethan extrahiert. Die vereinigten organischen Phasen werden über Natriumsulfat getrocknet und danach unter vermindertem Druck eingeengt. Der verbleibende Rückstand wird mit dem Laufmittel Cyclohexan: Ethylacetat = 2:1 an Kieselgel chromatographiert. Nach dem Eindampfen des Eluates erhält man 2,2 g (47% der Theorie) an 1-(4-Chlorphenyl)-2-(1-fluorcyclopropyl)-3-(1,2,4-triazol-1-yl)-propan-2-ol in Form einer Festsubstanz.
- ¹H-NMR (200 MHz, CDCl₃):: δ=0,0-0,8 (m, 4H); 3,0 (AB-System, 2H); 4,01 (s,1H, OH); 4,3 (AB-System, 2H); 7,28 (s,4H); 7, 98 (s, 1H); 8, 13 (s, 1H).

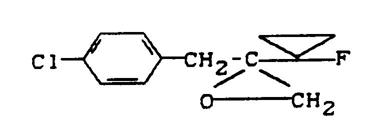

In ein Gemisch aus 2,2 g (17,1 mmol) Trimethylsulfoxoniumchlorid und 3.3 g (15,5 mmol) 4-Chlorbenzyl-(1-fluorcyclopropyl)-keton in 20 ml Toluol tropft man bei Raumtemperatur innerhalb einer Stunde 21 ml 45%ige wäßrige Natronlauge ein. Nach beendeter Zugabe wird 2 Stunden bei 40°C nachgerührt. Anschließend werden die Phasen getrennt, und die wäßrige Phase wird dreimal mit Toluol extrahiert. Die vereinigten organischen Phasen werden einmal mit Wasser gewaschen, über Natriumsulfat getrocknet und unter vermindertem Druck eingeengt. Der Rückstand wird ohne zusätzliche Reinigung für die weiter Umsetzung verwendet.

In den folgenden Verwendungsbeispielen wurde die Verbindung der nachstehend aufgeführten Formel als Vergleichssubstanz eingesetzt. 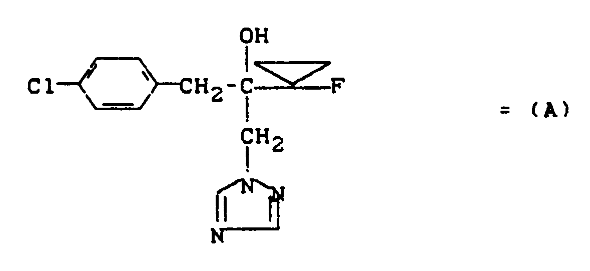
(Bekannt aus EP-OS 0 297 345)

### Beispiel A

### Erysiphe-Test (Gerste) / protektiv

- Lösungsmittel:: 100 Gew.-Teile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%.

### Beispiel B

### Erysiphe-Test (Weizen) / protektiv

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%, während die Vergleichssubstanz (A) einen Wirkungsgrad von 85% aufweist.

### Beispiel C

### Erysiphe-Test (Gerste) / kurativ

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20°C und einer relativen Luftfeuchtigkeit von ca. 80 % aufgestellt: um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%.

### Beispiel D

### Erysiphe-Test (Weizen) / kurativ

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%.

### Beispiel E

### Fusarium nivale (var. majus)-Test (Weizen) / protektiv

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Fusarium nivale (var. majus) besprüht.

Die Pflanzen werden in einem Gewächshaus unter lichtdurchlässigen Inkubationshauben bei einer Temperatur von ca. 15°C und einer relativen Luftfeuchtigkeit von ca. 100% aufgestellt.

4 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%, während die Vergleichssubstanz (A) einen Wirkungsgrad von 40% aufweist.

### Beispiel F

### Pyrenophora teres-Test (Gerste) / kurativ

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer Konidiensuspension von Pyrenophora teres besprüht. Die Pflanzen verbleiben 48 Stunden bei 20°C und 100% rel. Luftfeuchtigkeit in einer Inkubationskabine. Anschließend besprüht man die Pflanzen mit der Wirkstoffzubereitung taufeucht.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%.

### Beispiel G

### Leptosphaeria nodorum-Test (Weizen) / protektiv

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100 %.

### Beispiel H

### Gibberella zeae-Test (Gerste) / protektiv syn. Fusarium graminearum

- Lösungsmittel:: 100 Gew.-Teile Dimethylformamid
- Emulgator:: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Gibberella zeae besprüht.

Die Pflanzen werden in einem Gewächshaus unter lichtdurchlässigen Inkubationshauben bei einer Temperatur von ca. 20°C und einer relativen Luftfeuchtigkeit von ca. 100% aufgestellt.

4 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von 100%.

### Beispiel I

### Sclerotinia-Test (Buschbohne) / protektiv

- Lösungsmittel:: 4,7 Gewichtsteile Aceton
- Emulgator :: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden aufjedes Blatt 2 kleine mit Sclerotinia sclerotiorum bewachsene Agarstücke aufgelegt. Die inokulierten Pflanzen werden in einer abgedunkelten, feuchten Kammer bei 20°C aufgestellt. 3 Tage nach der Inokulation wird die Größe bei Befallsflecken auf den Blättern ausgewertt.

In diesem Test zeigt die erfindungsgemäße Verbindung (I-1) bei einer Wirkstoffkonzentration von 100 ppm in der Spritzflüssigkeit einen Wirkungsgrad von 100%.

### Beispiel K

### Sphaerotheca-Test (Gurke) / protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigt die erfindungsgemäße Verbindung (I-1) bei einer Wirkstoffkonzentration von 1 ppm in der Spritzflüssigkeit einen Wirkungsgrad 100%.

### Beispiel L

### Pyricularia-Test (Reis) / protektiv

- Lösungsmittel:: 12,5 Gewichtsanteile: Aceton
- Emulgator:: 0,3 Gewichtsanteile: Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

In diesem Test zeigt die erfindungsgemäße Verbindung (I-1) bei einer Konzentration von 0,025 % in der Spritzflüssigkeit einen Wirkungsgrad von 80%, während die Vergleichssubsstanz (A) keine Wirkung aufweist.

### Beispiel M

### Pyricularia-Test (Reis) / systemisch

- Lösungsmittel:: 12,5 Gewichtsteile: Aceton
- Emulgator:: 0,3 Gewichtsteile: Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

In diesem Test zeigt die erfindungsgemäße Verbindung (I-1) bei einer Aufwandmenge von 100 mg Wirkstoff pro 100 cm² einen Wirkungsgrad von 100%, während die Vergleichssubstanz (A) einen Wirkungsgrad von 50% aufweist.

### Beispiel N

### Pyrenophora teres-Test (Gerste) / protektiv

- Lösungsmittel:: 100 Gew.-Teile Dimethylformamid
- Emulgator:: 0,25 Gew.-Teile Alkyarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigt die erfindungsgemäße Verbindung (I-1) bei einer Konzentration von 250 ppm in der Spritzflüssigkeit einen Wirkungsgrad von über 80%, während die Vergleichssubstanz (A) einen Wirkungsgrad von 25% aufweist.

### Beispiel O

### Fusarium nivale (var. nivale) - Test (Weizen) / protektiv

- Lösungsmittel:: 100 Gewichtsteile Dimethylformamid
- Emulgator:: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbslages werden die Pflanzen mit einer Konidiensuspension von Fusarium nivale var. nivale besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt.

3 - 4 Tage nach der Inokulation erfolgt die Auswertung.

In diesem Text zeigt die erfindungsgemäße Verbindung (I-1) bei einer Konzentration von 250 ppm in der Spritzflüssigkeit einen Wirkungsgrad von 90%, während die Vergleichssubstanz (A) einen Wirkungsgrad von 30% aufweist.

## Patentansprüche

1. Azolylmethyl-fluorcyclopropyl-Derivate der Formel 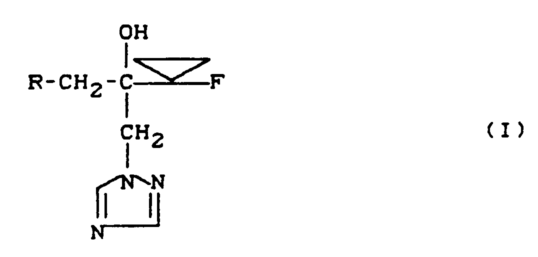 in welcher
R für 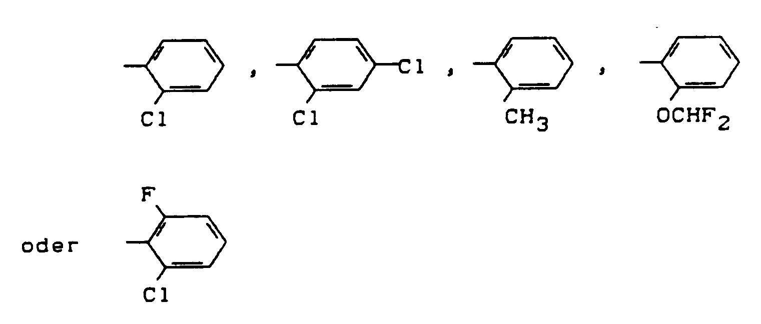 steht,
sowie deren Säureadditions-Salze und Metallsalz-Komplexe.

2. Verfahren zur Herstellung von Azolylmethyl-fluorcyclopropyl-Derivaten der Formel 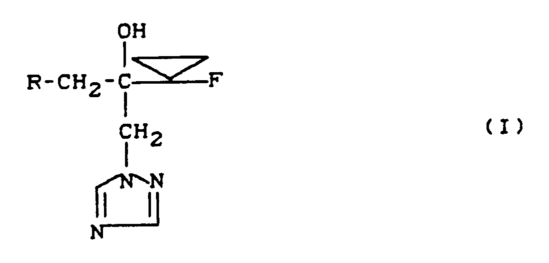 in welcher
R für 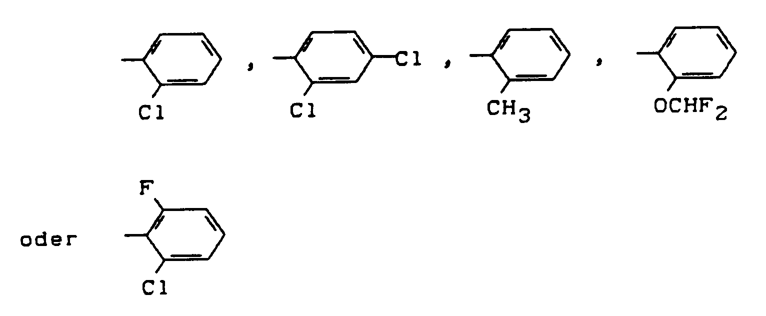 steht,
sowie von deren Säureadditions-Salzen und Metallsalz-Komplexen,
dadurch gekennzeichnet, daß man Oxirane der Formel 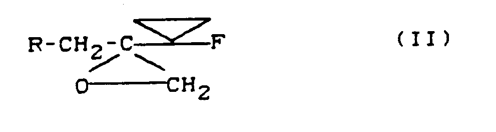 in welcher
R die oben angegebene Bedeutung hat,
mit 1,2,4-Triazol der Formel 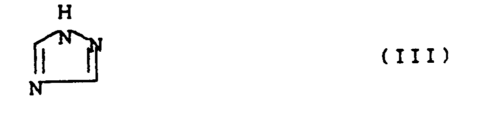 gegebenenfalls in Gegenwart eines Säurebindemittels und in Gegenwart eines Verdünnungsmittels umsetzt, und gegebenenfalls anschließend an die so erhaltenen Verbindungen der Formel (I) eine Säure oder ein Metallsalz addiert.

3. Fungizide Mittel, gekennzeichnet durch einen Gehalt an mindestens einem Azolylmethylfluorcyclopropyl-Derivat der Formel (I) gemäß Anspruch 1 beziehungsweise an einem Säureadditions-Salz oder Metallsalz-Komplex eines Azolylmethyl-fluorcyclopropyl-Derivates der Formel (I).

4. Verwendung von Azolylmethyl-fluorcyclopropyl-Derivaten der Formel (I) gemäß Anspruch 1 beziehungsweise von deren Säureadditions-Salzen und Metallsalz-Komplexen zur Bekämpfung von Pilzen.

5. Verfahren zur Bekämpfung von Pilzen, dadurch gekennzeichnet, daß man Azolylmethyl-fluorcyclopropyl-Derivate der Formel (I) gemäß Anspruch 1 beziehungsweise deren Säureadditions-Salze oder Metallsalz-Komplexe auf die Pilze und/oder deren Lebensraum ausbringt.

6. Verfahren zur Herstellung von fungiziden Mitteln, dadurch gekennzeichnet, daß man Azolylmethyl-fluorcyclopropyl-Derivate der Formel (I) gemäß Anspruch 1 beziehungsweise deren Metallsalz-Komplexe oder Säureadditions-Salze mit Streckmitteln und/oder oberflächenaktiven Stoffen vermischt.

7. Oxirane der Formel 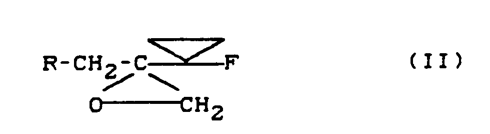 in welcher
R für 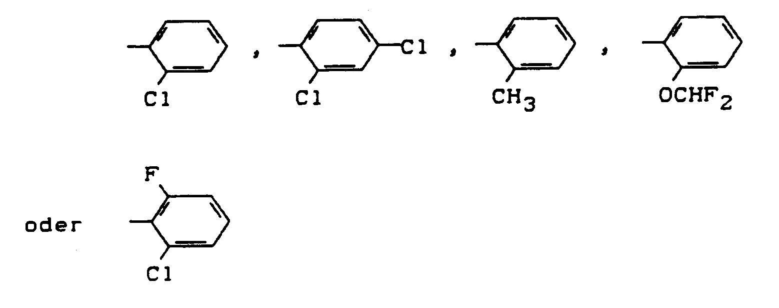 steht.

8. Verfahren zur Herstellung von Oxiranen der Formel 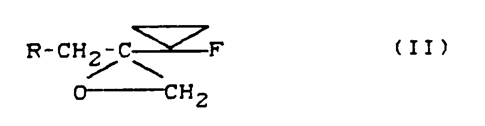 in welcher
R für 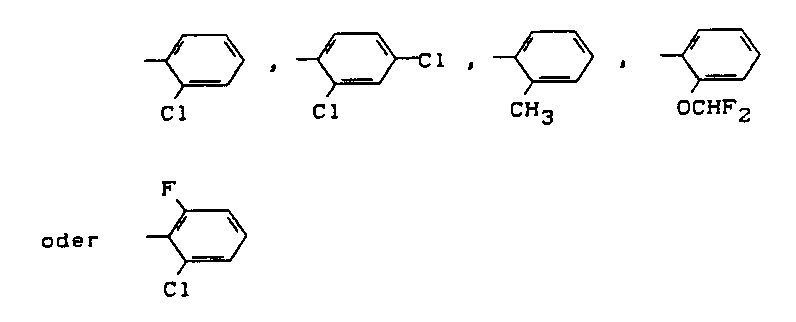 steht, dadurch gekennzeichnet, daß man Benzylketone der Formel 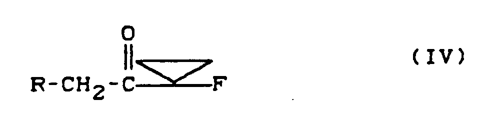 in welcher
R die oben angegebene Bedeutung hat,
entweder
α) mit Dimethyloxosulfonium-methylid der Formel 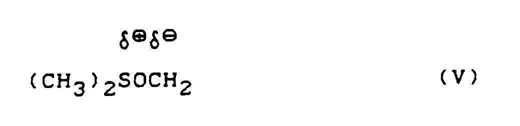 oder
β) mit Dimethylsulfonium-methylid der Formel 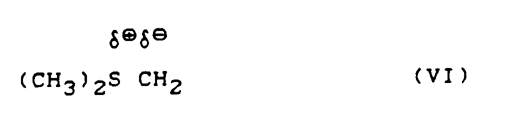 in Gegenwart eines Verdünnungsmittels umsetzt.

## Claims

1. Azolylmethyl-fluorocyclopropyl derivatives of the formula 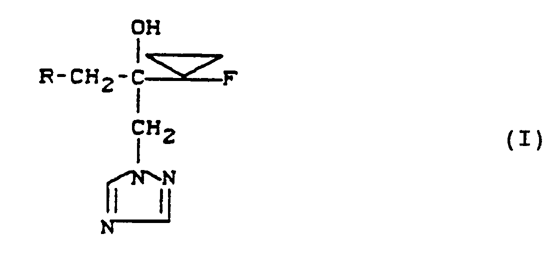 in which
R represents 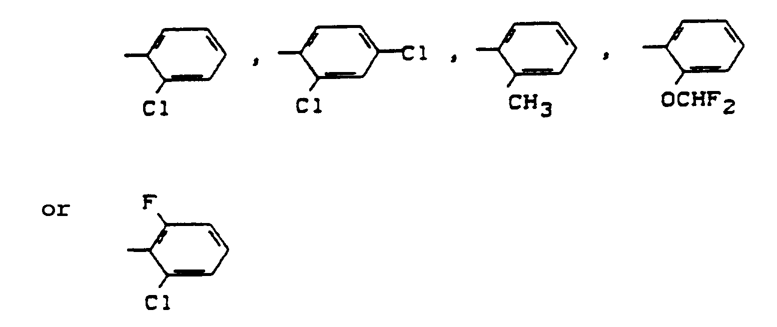 and their acid addition salts and metal salt complexes.

2. Process for the preparation of azolylmethylfluorocyclopropyl derivatives of the formula 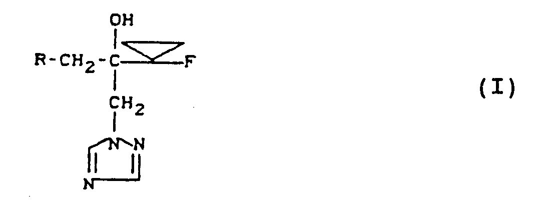 in which
R represents 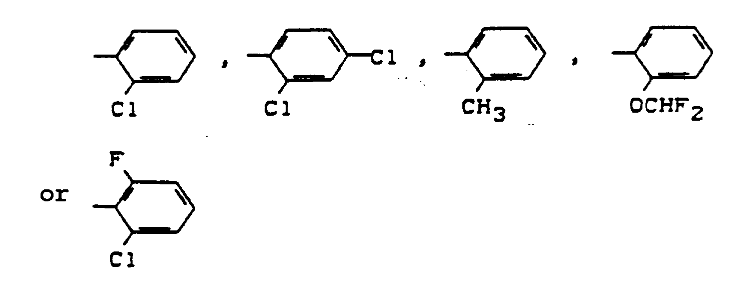 and their acid addition salts and metal salt complexes, characterized in that oxiranes of the formula 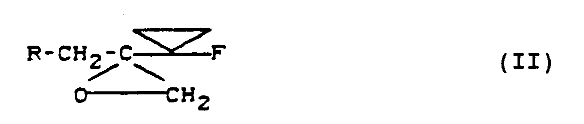 in which
R has the abovementioned meaning,
are reacted with 1,2,4-triazole, of the formula 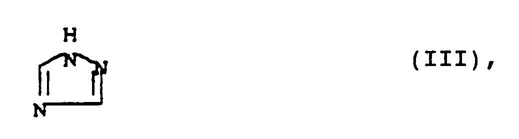 if appropriate in the presence of an acid-binding agent and in the presence of a diluent, and, if appropriate, an acid or a metal salt are subsequently added onto the resulting compounds of the formula (I).

3. Fungicidal agents, characterized in that they contain at least one azolylmethyl-fluorocyclopropyl derivative of the formula (I) according to Claim 1, or an acid addition salt or metal salt complex of an azolylmethyl-fluorocyclopropyl derivative of the formula (I).

4. Use of azolylmethyl-fluorocyclopropyl derivatives of the formula (I) according to Claim 1 or of their acid addition salts and metal salt complexes for combating fungi.

5. Method of combating fungi, characterized in that azolylmethyl-fluorocyclopropyl derivatives of the formula (I) according to Claim 1 or their acid addition salts or metal salt complexes are applied to the fungi and/or their environment.

6. Process for the preparation of fungicidal agents, characterized in that azolylmethyl-fluorocyclopropyl derivatives of the formula (I) according to Claim 1 or their metal salt complexes or acid addition salts are mixed with extenders and/or surface-active substances.

7. Oxiranes of the formula 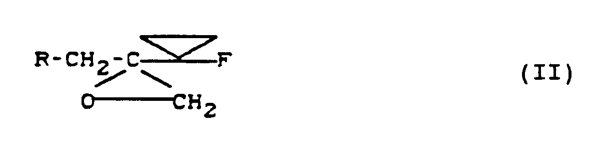 in which
R represents

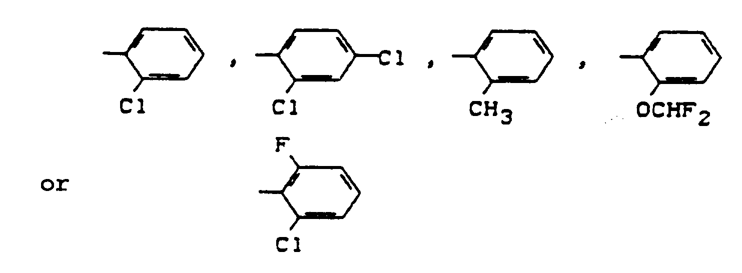

8. Process for the preparation of oxiranes of the formula 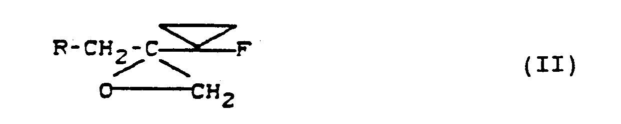 in which
R represents 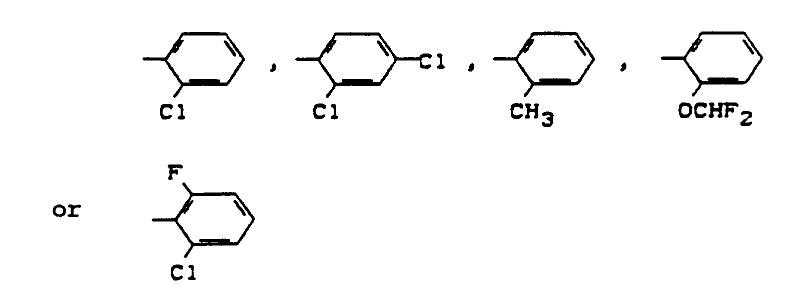 characterized in that benzyl ketones of the formula 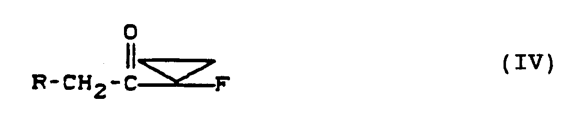 in which
R has the abovementioned meaning,
are reacted either
α) with dimethyloxosulphonium methylide, of the formula 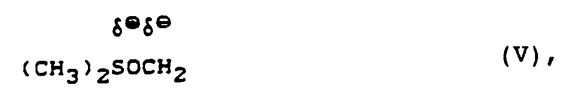 or
β) with dimethylsulphonium methylide, of the formula 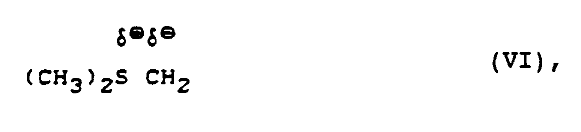 in the presence of a diluent.

## Revendications

1. Dérivés azolylméthyl-fluorocyclopropyliques de formule 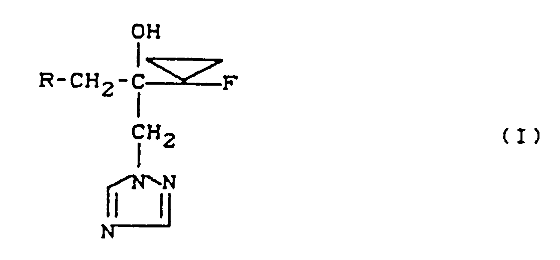 dans laquelle
R représente 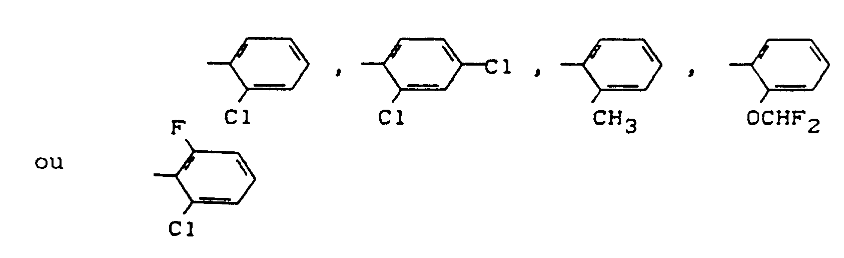 ainsi que leurs sels d'addition d'acides et leurs complexes de sels métalliques.

2. Procédé de production de dérivés azolylméthylfluorocyclopropyliques de formule 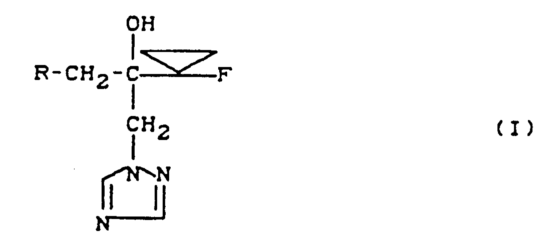 dans laquelle
R représente 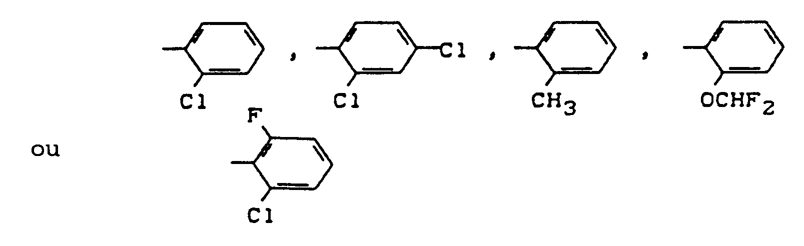 ainsi que de leurs sels d'addition d'acides et de leurs complexes de sels métalliques, caractérisé en ce qu'on fait réagir des oxirannes de formule 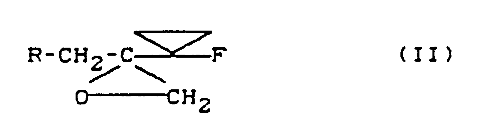 dans laquelle R a la définition indiquée ci-dessus,
avec le 1,2,4-triazole de formule 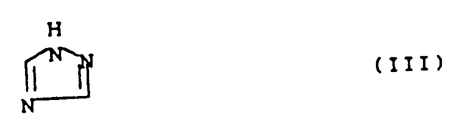 le cas échéant en présence d'un accepteur d'acide et en présence d'un diluant, puis on additionne ensuite le cas échéant un acide ou un sel métallique sur les composés de formule (I) ainsi obtenus.

3. Composition fongicide, caractérisée par une teneur en au moins un dérivé azolylméthylfluorocyclopropylique de formule (I) suivant la revendication 1 ou respectivement en au moins un sel d'addition d'acide ou un complexe de sel métallique d'un dérivé azolylméthylfluorocyclopropylique de formule (I).

4. Utilisation de dérivés azolylméthylfluorocyclopropyliques de formule (I) suivant la revendication 1 ou respectivement de leurs sels d'addition d'acides et de leurs complexes de sels métalliques pour combattre des champignons.

5. Procédé pour combattre des champignons, caractérisé en ce qu'on épand des dérivés azolylméthylfluorocyclopropyliques de formule (I) suivant la revendication 1 ou respectivement leurs sels d'addition d'acides ou leurs complexes de sels métalliques sur les champignons et/ou sur leur milieu.

6. Procédé de préparation de compositions fongicides, caractérisé en ce qu'on mélange des dérivés azolylméthyl-fluorocyclopropyliques de formule (I) suivant la revendication 1 ou respectivement leurs complexes de sels métalliques ou leurs sels d'addition d'acides avec des diluants et/ou des agents tensioactifs.

7. Oxirannes de formule 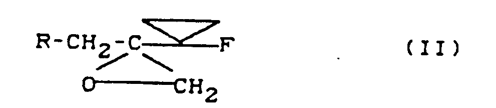 dans laquelle
R représente

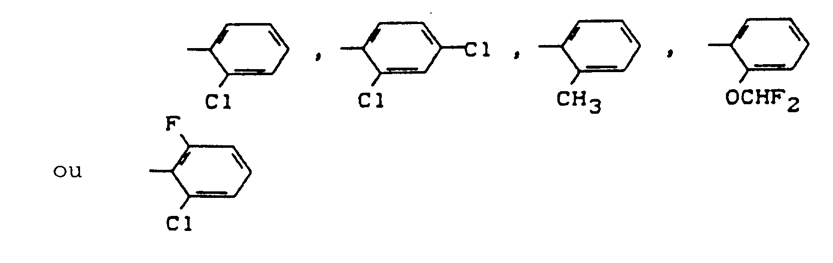

8. Procédé de production d'oxirannes de formule 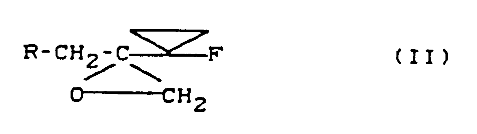 dans laquelle
R représente 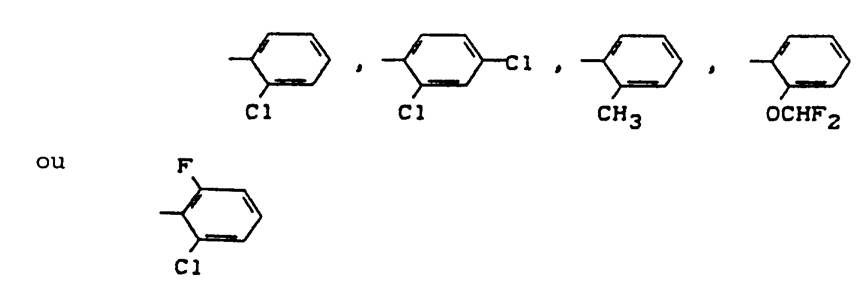 caractérisé en ce qu'on fait réagir des benzylcétones de formule 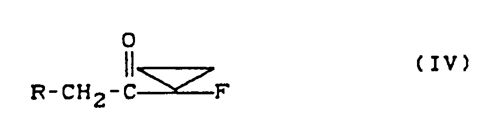 dans laquelle
R a la définition indiquée ci-dessus,
α) avec du méthylure de diméthyloxosulfonium de formule 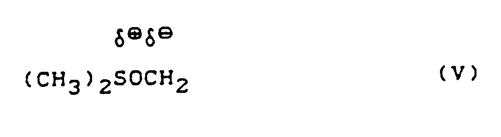 ou
β) du méthylure de diméthylsulfonium de formule 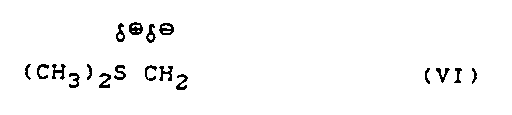 en présence d'un diluant.
